# FASCICULE DE BREVET EUROPEEN

(11) **EP 2 196 395 B1**
(45) Date de publication et mention de la délivrance du brevet: **09.11.2011**
(21) Numéro de dépôt: 08171045.1
(22) Date de dépôt: 09.12.2008
(51) Int. Cl.: B65B 5/08, B65B 35/36, B65B 35/44, B65G 47/08, B65G 47/84

(54) **Dispositif et procédé de conditionnement de seringues en nests**
Vorrichtung und Verfahren zur Verpackung von Spritzen in Nestern
Device and method for packaging nested syringes

(43) Date de publication de la demande: 16.06.2010
(73) Titulaire: Sybermat, 1420 Braine-l'Alleud (BE)
(72) Inventeur: Van Roy, Pierre Jaques, 1350 Jandrain (BE)
(74) Mandataire: Claeys, Pierre

(56) Documents cités:
- EP-A- 0 092 749
- EP-A- 0 979 787
- EP-A- 0 994 025
- DE-A1- 10 318 731
- DE-A1- 19 604 100
- DE-C1- 4 307 906

## Description

La présente invention se rapporte à un dispositif de conditionnement de seringues en nests comprenant :
- des moyens de mise au pas des seringues, agencés pour séparer et écarter audit pas les seringues amenées,
- des premiers moyens de préhension desdites seringues mises audit pas, présentant une position de prise dans laquelle les moyens de préhension saisissent une quantité prédéterminée de seringues et une position de libération dans laquelle les moyens de préhension relâchent ladite quantité prédéterminée de seringues pour les disposer dans des nests.

Les seringues sont communément emballées dans des présentoirs qui maintiennent les seringues de manière ordonnée et espacée l'une de l'autre pour éviter les contacts entre les seringues, en particulier pendant le transport.

Les nests sont des présentoirs à seringues, généralement en polypropylène. Les nests comprennent une plaque basale munie de tubes s'étendant tous dans une même direction au travers desquels les seringues sont enfilées et présentent une hauteur h. Les seringues présentent à une extrémité, opposée à l'orifice d'injection auquel sera fixée éventuellement une aiguille, une collerette d'un diamètre supérieur à celui de la seringue. Lorsque les seringues sont enfilées dans les tubes, les collerettes reposent sur l'extrémité de ces tubes qui est libre, c'est-à-dire non reliée à la plaque basale.

Les seringues sont communément commercialisées vides dans cette conformation, et souvent, sans le piston, qui ne sera apposé qu'après remplissage de la seringue.

Les sociétés productrices de seringues nécessitent donc un dispositif permettant de conditionner les seringues fabriquées dans les nests afin de pouvoir les livrer avec le moins de casse possible. Si une seringue cassée ne représente pas un réel trou financier pour le producteur, la possible présence d'éclats de verre minuscules, voire microscopiques, dans les autres seringues est un problème majeur car le risque de provoquer de sérieux troubles chez le patient auquel cette fraction microscopique de verre aura été injectée est plus que présent. Il est donc clair que les fabriquant de seringues doivent réduire le risque de la présence de ces éclats et cette considération occupe une place très importante dans le conditionnement des seringues.

De plus, lorsqu'une société pharmaceutique souhaite conditionner un produit dans une seringue, en particulier dans une seringue en verre, elle s'adresse à des fabricants de seringues et elles sont livrées en présentoirs (nests). Généralement les nests sont munis d'un capot de protection qui protège les seringues de la casse, les seringues étant disposées parallèlement et maintenues substantiellement écartées l'une de l'autre justement grâce auxdits nests. Toutefois, pour les remplir, les seringues peuvent être dénestées et convoyées sur une bande transporteuse ou elle sont alors remplies, munies de leur piston, autoclavées et ensuite convoyées à nouveau pour un reconditionnement en nests. Bien entendu, pour cette étape également, le risque de la présence d'un éclat de verre est à réduire autant que ce peut et dès lors, il est crucial de réduire le risque de casse des seringues.

Dans les dispositifs de nestage connus, les seringues parviennent sur une bande transporteuse à l'endroit où elles doivent être placées dans des nests. Un système de direction permet de les orienter et de les espacer d'une distance appropriée l'une de l'autre comme une vis de mise au pas. Ensuite, une pince robotisée en prélève une ou plusieurs simultanément, les présente au dessus des orifices du nest et les lâche. De cette façon, les seringues sont nestées ou renestées après remplissage.

Malheureusement, un tel dispositif de conditionnement de seringues en nests nécessite un arrêt de la vis à pas à chaque fois que la pince robotisée doit prélever des seringues. Tout arrêt et redémarrage, quoique coûteux en terme d'énergie est en plus une source potentielle de dégradation des seringues. En effet, le convoyage des seringues est chaque fois arrêté brutalement en entrechoquant les seringues les unes contre les autres en guise d'arrêt. Dans le cas de bris, même si les seringues ne sont pas complètement brisées, des éclats de verre se forment et augmentent le risque de trouver un éclat de verre dans le contenu d'une seringue.

L'invention a pour but de pallier les inconvénients de l'état de la technique en procurant un dispositif de conditionnement de seringues en nests permettant de ne pas devoir arrêter le système de direction qui permet de les orienter et de les espacer d'une distance appropriée l'une de l'autre. En plus, le dispositif selon l'invention permet d'atteindre des rendements de conditionnement de 2 à 3 fois plus élevés. En effet, le fait de ne plus être limité par le risque de casse permet d'augmenter le nombre de seringues conditionnées à l'heure.

Pour résoudre ce problème, il est prévu suivant l'invention, un dispositif tel qu'indiqué au début comprenant en outre une chaîne de transport à maillons présentant ledit pas, en circuit fermé et en mouvement au moins autour de deux axes d'entrainement, agencée pour transporter et mettre en mouvement les seringues provenant des moyens de mise au pas, chaque maillon étant agencé pour loger au moins une partie d'une seringue, et en ce que lesdits premiers moyens de préhension passent de la position de libération à la position de prise, en mouvement synchronisé avec le mouvement de la chaîne, pour prélever une quantité prédéterminée de seringues en mouvement à partir de ladite chaîne.

De cette façon, les seringues espacées régulièrement dudit pas par lesdits moyens de mise au pas sont ensuite transportées chacune dans un maillon d'une chaîne à maillon qui les convoie sans temps d'arrêt des moyens de mise au pas. Les moyens de préhension en mouvement synchronisé avec la chaîne, accostent la chaîne de transport et saisissent lesdites seringues en mouvement. Il n'y a donc aucun moment pendant lequel la chaîne ou les moyens de mise au pas sont arrêtés. Une fois les seringues saisies par les moyens de préhension, c'est-à-dire, un fois que les moyens de préhension sont dans la position de prise, les moyens de préhension s'écartent de la chaîne et se dirigent vers le nest où les seringues seront libérées desdits moyens de préhension et donc conditionnées dans les tubes en saillie de la plaque basale dudit nest et les collerettes desdites seringues reposeront sur l'extrémité libre desdits tubes. Les collerettes desdites seringues servant d'arrêt à la pénétration des seringues dans les tubes. De manière très surprenante, on peut atteindre selon l'invention des rendements de conditionnement de 30000 seringues par heure alors qu'avec les dispositifs connus, on atteignait à peine 12000 seringues par heure tout en risquant d'avoir des éclats dans les seringues. Il va donc de soi que le dispositif selon l'invention représente une amélioration significative et particulièrement inventive des dispositifs connus.

Avantageusement, le dispositif de conditionnement de seringues selon l'invention comprend une roue de transfert agencée pour charger les seringues mises au pas par lesdits moyens de mise au pas dans ladite chaîne de transport.

De cette façon, la continuité du transport entre les moyens de mise au pas et la chaîne de transport est améliorée, ce qui réduit encore le risque de casse et augmente encore la rentabilité du dispositif selon l'invention. En outre, une telle roue de transfert permet un changement d'axe lorsque c'est nécessaire entre la direction de la mise au pas et la direction du transfert dans la chaîne de transport, ce qui peut dans certains cas, lorsque c'est nécessaire réduire l'encombrement du dispositif selon l'invention, dans des endroits comme les salles blanches où cela est particulièrement essentiel car tout espace à entretenir est très coûteux.

Dans une forme de réalisation particulière, lesdits maillons de la chaîne sont reliés entre eux par un point de pivot permettant aux maillons d'être mobiles l'un par rapport à l'autre de manière unidirectionnelle à partir d'un axe de chaîne passant par chaque point de pivot.

Par les termes "de manière unidirectionnelle", on entend que, par rapport à la direction d'avancée de la chaîne de transport, les maillons peuvent entrer en rotation autour dudit point de pivot d'un côté seulement, c'est-à-dire, puisque la chaîne est en circuit fermé, uniquement vers l'intérieur de la forme réalisée par le circuit fermé ou uniquement vers l'extérieur de celui-ci. Dès lors, pour réaliser ceci, le maillon présente par exemple une butée qui bloque la rotation du maillon dans un sens par rapport à la direction d'avancée de la chaîne. Cette butée peut être, à titre d'exemple, un élément en saillie ou une paroi externe qui comporte une arrête.

Il ressort clairement de ceci que la chaîne peut former n'importe quelle forme, que nous appellerons forme simple telles qu'une ellipse, un cercle, un rectangle, un carré, un trapèze, un hexagone, un octogone ou analogue.

Dans une variante particulière, lesdits maillons de la chaîne sont reliés entre eux par un point de pivot permettant aux maillons d'être mobiles l'un par rapport à l'autre de manière bidirectionnelle à partir d'un axe de chaîne passant par chaque point de pivot.

Par les termes "de manière bidirectionnelle", on entend que, par rapport à la direction d'avancée de la chaîne de transport, les maillons peuvent entrer en rotation autour dudit point de pivot de part et d'autre, c'est-à-dire, puisque la chaîne est en circuit fermé, vers l'intérieur de la forme réalisée par le circuit fermé et vers l'extérieur de celui-ci. Dès lors, pour réaliser ceci, le maillon présente de préférence, une paroi externe arrondie qui permet la rotation du maillon dans les deux sens par rapport à la direction d'avancée de la chaîne.

Il ressort clairement de ceci que la chaîne peut former n'importe quelle forme, que nous appellerons forme complexe, par exemple une forme fermée comprenant un ou plusieurs tronçon en S.

Bien entendu, toutes les combinaisons sont possibles, par exemple, le point de pivot permettant aux maillons d'être mobiles l'un par rapport à l'autre de manière bidirectionnelle peut également être utilisé pour une chaîne circulant en circuit fermé de forme simple au sens présenté ci-dessus, par exemple pour uniformiser la fabrication de ces chaînes et ne pas devoir fabriquer deux chaînes différentes pour des circuits de formes simples ou complexes.

De préférence, chaque maillon présente une section sensiblement identique à la section de la seringue à conditionner. Dès lors, la seringue est logée de manière ajustée dans ledit maillon, ce qui évite tout jeu au sein de celui-ci. Ceci représente un atout considérable car il permet d'utiliser une vitesse maximale de déplacement de la chaîne sans risque de casse, puisque les seringues ne bougent pas dans celle-ci. De plus, les moyens de préhension peuvent être optimalisés car la position à laquelle ils vont saisir les seringues sera prédéterminée et ne devra pas forcément admettre un écart de position pour le cas où les seringues auraient bougé. Il ressort clairement que dans un dispositif selon l'invention qui permet le conditionnement de seringues qui sont des objets de petite taille et qui sont fragiles et dans un monde où tout doit être particulièrement propre et exempt d'éclat de verre, une telle précision est un atout considérable.

Avantageusement, chaque maillon agencé pour loger au moins une partie d'une seringue comprend une ouverture latérale d'entrée ou de sortie de la seringue faisant face à une seringue mise au pas par lesdits des moyens de mise au pas.

Pour des raisons de fluidité de mouvement, il est préférable que les seringues passent des moyens de mise au pas (via la roue de transfert ou non) au maillon destiné à les loger par une ouverture latérale, plutôt que par exemple par l'ouverture supérieure de celui-ci.

De préférence, ladite ouverture présente une largeur ajustée par rapport à la section de la seringue à loger dans ledit maillon. En effet, lorsque la largeur de l'ouverture est sensiblement identique au diamètre de la seringue, le dispositif selon l'invention permet de former un passage ajusté et de réduire au maximum le jeu entre la seringue et le maillon et permet donc de réduire le risque d'échappement fortuit de la seringue du maillon.

Dans une forme de réalisation particulière, chaque maillon présente une surface supérieure munie d'une saillie agencée pour bloquer ladite seringue logée dans ledit maillon. Un effet, dans un cas préférentiel, la saillie se trouve au sommet de l'ouverture du maillon. De cette façon, lorsque la seringue passe au travers de l'ouverture, elle repose sur la saillie. Lorsque la seringue est complètement logée dans le maillon, elle repose sur l'arrête supérieure de celui-ci et se trouve donc à une altitude plus basse que lorsqu'elle reposait sur la saillie. Elle est donc empêchée de tout mouvement vers l'ouverture et donc un échappement fortuit est très peu probable. Ceci améliore la précision du dispositif selon l'invention encore un peu plus.

L'invention comprend avantageusement en outre, un convoyeur de préférence externe à ladite chaîne de transport en circuit fermé pour le transport des nests à conditionner ou conditionnés.

Dans une forme de réalisation particulièrement avantageuse du dispositif selon l'invention, lesdits moyens de mise au pas sont alimentés par des moyens d'amenée des seringues, en particulier par un convoyeur ou une bande transporteuse.

Selon l'invention, lesdits moyens de mise au pas présentent un pas homogène. Dans une variante, ils présentent un pas variable, en fonction du type de convoyeur utilisé en amont du dispositif selon l'invention. Si les seringues arrivent déjà espacées régulièrement l'une de l'autre, des moyens de mises au pas pourront comporter un pas homogène. Si les seringues arrivent serrées l'une par rapport à l'autre, de préférence, les moyens de mise au pas comporteront un pas variable qui permettra en fin de course des moyens de mise au pas d'espacer les seringues au pas de la chaîne ou de la roue de transfert, c'est-à-dire au pas souhaité.

En outre, la roue de transfert et son moyen de mise au pas peuvent être remplacés par un moyen de mise au pas variable couplé à un galet de renvoi de chaîne qui bien que plus complexe est une alternative envisagée selon l'invention.

De plus, dans une forme de réalisation particulière, le dispositif selon l'invention comporte des deuxièmes moyens de préhension desdites seringues mises audit pas, présentant une position de prise dans laquelle les deuxièmes moyens de préhension saisissent une quantité prédéterminée de seringues et une position de libération dans laquelle les deuxièmes moyens de préhension relâchent ladite quantité prédéterminée de seringues pour les disposer dans des nests, lesdits deuxièmes moyens de préhension passant de la position de libération à la position de prise, en mouvement synchronisé avec le mouvement de la chaîne, pour prélever une quantité prédéterminée de seringues en mouvement à partir de ladite chaîne.

Dans cette forme de réalisation avantageuse, les deuxièmes moyens de préhension permettent de traiter le double de seringues par heures. Les deuxièmes moyens de préhension peuvent bien entendu soit travailler en synchronisation avec les premiers moyens de préhension, mais aussi de manière décalée.

Les premiers et deuxièmes moyens de préhension sont prévus pour saisir et libérer un lot de X seringue qui peut être de 6, 7, 8, 9, 10, 11, 12 ou tout autre nombre adéquat. De préférence, les moyens de préhension peuvent saisir autant de seringues qu'il y a de tubes dans une rangée d'un nest.

Lorsque les premiers et deuxièmes moyens de préhension travaillent en synchronisation, ils accostent la chaîne ensemble, chacun à une position prédéterminée à laquelle un lot de seringue complet se trouve, ils saisissent en même temps, chacun leur propre lot de seringue, et quittent et se dirigent vers le nest ensemble pour y libérer leur seringues.

Dans une forme particulière de l'invention, les premiers moyens de préhension et les deuxièmes moyens de préhension se rassemblent l'un à proximité de l'autre et un fois au-dessus du nest, les moyens de préhension libèrent les seringues dans les tubes destinés à les recevoir. Les premiers moyens et deuxièmes moyens de préhension rassemblés permettent alors de conditionner les seringues dans deux rangées consécutives, ou dans deux rangées espacées l'une de l'autre par une rangée vide qui sera conditionnée ultérieurement par l'arrivage suivant.

Il est bien entendu que les premiers et deuxième moyens de préhension peuvent également ne pas se rassembler ou se rassembler plus ou moins fort, par exemple si l'on souhaite remplir des rangées plus espacées l'une de l'autre. Dans ce cas, pour un nest comportant 20 rangées, il peut être souhaitable que les premiers moyens de préhension conditionnent la rangée 1 et les deuxièmes moyens de préhension conditionnent la rangée 10, ensuite, respectivement la rangée 2 et 11, et puis, respectivement la rangée 3 et 12, et ainsi de suite.

Lorsque les premiers et deuxièmes moyens de préhension travaillent de manière décalée, les premiers moyens de préhension peuvent accoster la chaîne et saisir les seringues pendant que les deuxièmes moyens de préhension quittent la chaîne et se dirigent vers les nest pour y lâcher les seringues saisies et transportées. Ensuite, les premiers moyens de préhension quittent la chaîne et se dirigent vers les nest pour y lâcher les seringues saisies et transportées pendant que les deuxièmes moyens de préhension accostent la chaîne et saisissent les seringues.

En particulier, dans une variante de l'invention, le nombre d'axes d'entrainement vaut au moins 4 et dans lequel ladite chaîne comprend plus d'un tronçon aller et plus d'un tronçon retour.

Par " plus d'un tronçon aller et plus d'un tronçon retour", on entend que la chaîne forme au moins un S. Bien entendu, le sens d'avancée des seringues est toujours celui de la chaîne, mais par rapport à deux points fixes, la direction est inversée entre ledit tronçon aller et ledit tronçon retour.

Dans cette variante avantageuse, lesdits premiers et deuxièmes moyens de préhension sont synchronisés et passent de la position de libération à la position de prise et saisissent lesdites seringues ensemble et les libèrent dans le nest ensemble, lesdits premiers et deuxièmes moyens de préhension étant agencés pour prélever lesdites seringues en mouvement de la chaîne, chacun sur un tronçon aller différent ou chacun sur un tronçon retour différent.

Dans cette forme de réalisation avantageuse, les moyens de préhension accostent à des endroits différents, mais sur des tronçons où, par rapport à deux points fixes, la direction d'avancée est identique. Dans ce cas, si l'on considère que les premiers et deuxièmes moyens de préhension comportent un côté de préhension et un côté fermé, et que l'axe de prise va du côté fermé au côté de préhension, l'axe de prise des premiers moyens de préhension et celui des deuxièmes moyens de préhension sont alignés. Dès lors, les deuxièmes moyens de préhension et les premiers moyens de préhension travaillent avec la même orientation.

Dans une autre forme de réalisation, lesdits premiers et deuxièmes moyens de préhension sont synchronisés et passent de la position de libération à la position de prise et saisissent lesdites seringues ensemble et les libèrent dans le nest ensemble, lesdits premiers et deuxièmes moyens de préhension étant agencés pour prélever lesdites seringues en mouvement de la chaîne, l'un sur un tronçon aller et l'autre sur un tronçon retour. Dans ce cas, l'axe de prise des premiers moyens de préhension et celui des deuxièmes moyens de préhension sont inversés et les deuxièmes moyens de préhension et les premiers moyens de préhension travaillent avec une orientation inversée.

Avantageusement, le dispositif de conditionnement de seringues selon l'invention comprend un dispositif d'éjection de seringues en fin de circuit qui permet entre autres, lorsque l'on arrête la chaîne, en fin d'opération de pouvoir la vider des seringues dans les maillons mais non encore conditionnées en nest.

En outre, lorsqu'une seringue est défectueuse ou qu'elle a été brisée, ou qu'un des maillons n'est pas occupé par une seringues, lesdits moyens de préhension détectent avantageusement que le lot n'est pas complet. Dans ce cas, ils sont par exemple programmés pour ne pas prélever ce lot là et attendre le suivant. Dans un tel cas, il est préférentiel que le dispositif comprenne le moyen d'éjection de seringues susdit afin de vider les maillons encore occupés en fin de circuit. En outre, cela évite de devoir, réguler l'entrée des seringues dans la chaîne de transport qui conduirait inévitablement à un arrêt de la roue de transfert ou des moyens de mises au pas, ce qui aurait pour conséquence un entrechoquement, comme mentionné ci-avant et la casse qui y est généralement associée.

Dans une forme de réalisation avantageuse, lesdits moyens de mise au pas des seringues, ladite chaîne de transport à maillons présentant ledit pas, ladite roue de transfert et éventuellement ledit dispositif d'éjection sont agencés sur une plate-forme interchangeable.

Dans ce cas, lorsque le dispositif de conditionnement selon l'invention doit conditionner une autre taille de seringue, par exemple de diamètre inférieur, il ne faut pas remplacer le dispositif, mais uniquement la plate forme et cette opération ne prend que quelques minutes, plutôt que de devoir positionner de nouveaux éléments séparément.

D'autres formes de réalisation du dispositif suivant l'invention sont indiquées dans les revendications annexées.

L'invention a aussi pour objet un procédé comprenant les étapes de:
- amenée et mise au pas des seringues à conditionner dans une zone de mise au pas avec une obtention de seringues mise au pas,
- une préhension des seringues mises au pas à l'aide de moyens de préhension,
- un déplacement des seringues prélevées vers lesdits nests dans lesquels lesdites seringues sont à conditionner par les moyens de préhension.

Ce procédé est **caractérisé en ce qu'**il comprend en outre un transport par une chaîne, des seringues entre ladite zone de mise au pas et une zone de prélèvement et en ce que le prélèvement par les moyens de préhension est effectué en mouvement et comprend, de manière cyclique, une phase d'accostage des moyens de préhension sur ladite chaîne, une phase de prise et une phase de déplacement des seringues prélevées vers lesdits nests avec libération des seringues dans le nest susdit.

D'autres formes de réalisation du procédé suivant l'invention sont indiquées dans les revendications annexées.

D'autres caractéristiques, détails et avantages de l'invention ressortiront de la description donnée ci-après, à titre non limitatif et en faisant référence aux dessins annexés.
La figure 1 est une vue en perspective d'un nest comportant 100 seringues.
La figure 2A est une de profil d'une forme de réalisation particulièrement simple du dispositif de conditionnement de seringues selon l'invention.
La figure 2B est une vue d'en haut de la forme de réalisation illustrée à la figure 2A.
Les figures 3A, B et C illustrent la forme de réalisation illustrée aux figures 2 en fonctionnement, c'est-à-dire pendant les trois phases du cycle des moyens de préhension, à savoir, la phase d'accostage, la phase de prise et la phase de libération.
La figure 4A est une vue d'en haut d'une partie de la chaîne de transport à maillons présentant ledit pas entraînée par un des au moins deux axes d'entrainement.
La figure 4B est une vue d'en haut d'un maillon de ladite chaîne de transport.
La figure 4C est une vue de face d'un maillon de ladite chaîne de transport.
La figure 4D est une vue de côté d'un maillon de ladite chaîne de transport.
La figure 4E est une vue de face d'un maillon de ladite chaîne de transport contenant une seringue.
La figure 4F est une vue de face d'un ensemble de maillon des ladite chaîne de transport.
La figure 5 est une vue d'en haut d'une variante selon l'invention illustrant un point de pivot à maillons mobiles de manière bidirectionnelle.
La figure 6A est une vue de profil d'une forme de réalisation particulière du dispositif de conditionnement de seringues selon l'invention.
La figure 6B est une vue d'en haut de la forme de réalisation particulière du dispositif de conditionnement de seringues illustré à la figure 6A, dans lequel ladite chaîne comprend deux tronçons aller et deux tronçons retour.
Les figures 7A à 7C sont des vues d'en haut de la forme de réalisation illustrée à la figure 6A ou à la figure 6B, illustrée en fonctionnement et représentant les trois phases du cycle des moyens de préhension, la phase d'accostage, la phase de prise et la phase de libération avec conditionnement dans ledit nest.
Les figures 8A à 8C sont des vues d'en haut d'une variante de la forme de réalisation illustrée à la figure 6A ou à la figure 6B, illustrée en fonctionnement et représentant les trois phases du cycle des moyens de préhension, la phase d'accostage, la phase de prise et la phase de libération avec conditionnement dans ledit nest, dans laquelle les moyens de préhension saisissent les seringues du côté opposé.

Sur les figures, les éléments identiques ou analogues portent les mêmes références.

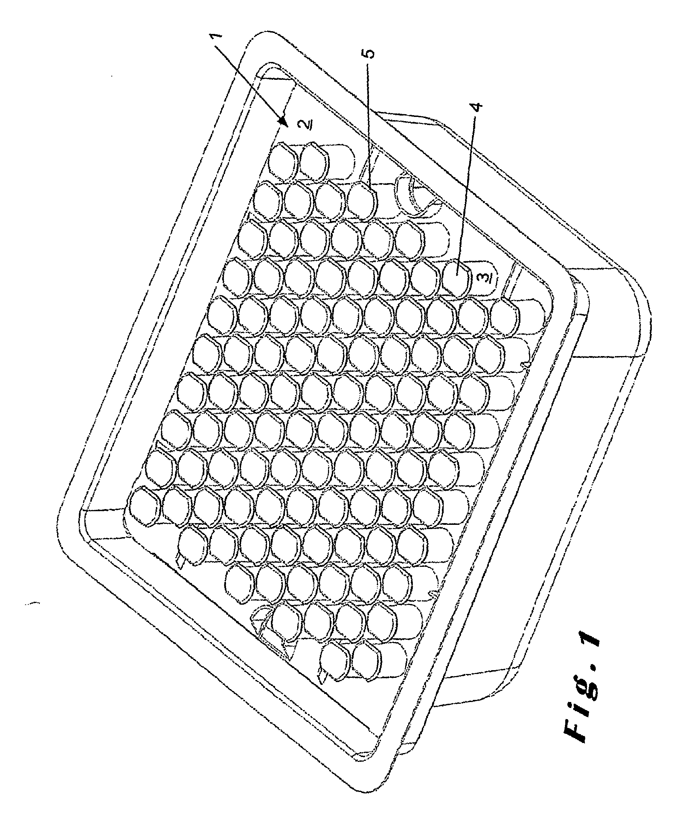

Comme on peut le voir à la figure 1, un nest 1 est un présentoir à seringues qui comprend une plaque basale 2 munie de tubes 3 s'étendant tous dans une direction perpendiculairement à la plaque basale 2 au travers desquels les seringues 4 sont enfilées. Les collerettes 5 des seringues 4 reposent alors sur les extrémités des tubes 3 qui sont opposées à la plaque basale 2.

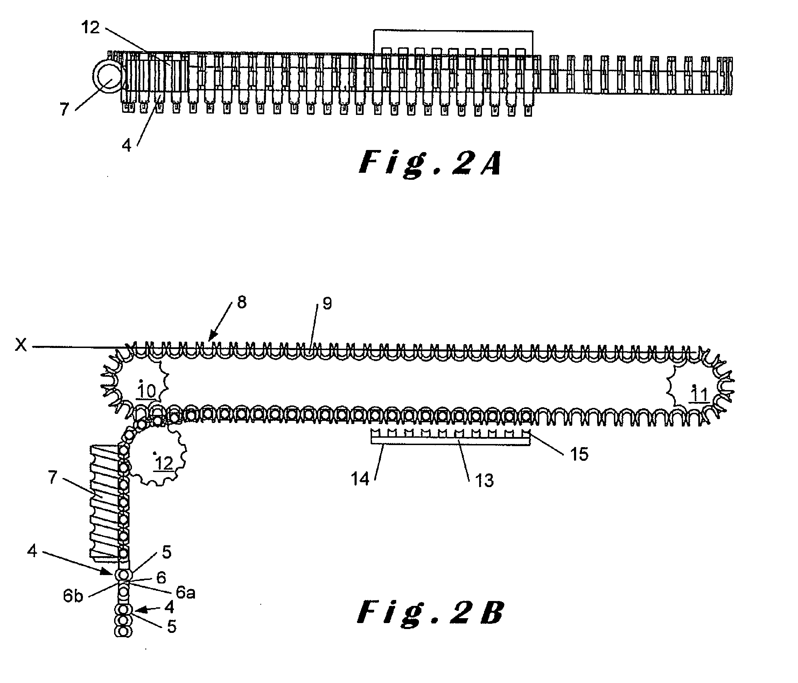

Les figures 2A et 2B illustrent des formes de réalisations particulièrement simplifiées du dispositif de conditionnement de seringues dans des nests.

Les seringues fabriquées ou conditionnées sont amenées à titre d'exemple par une bande transporteuse 6 vers le dispositif selon l'invention. Dans l'illustration ici, les seringues 4 sont positionnées collerettes 5 vers le haut et les collerettes 5 reposent sur deux parois parallèles 6a et 6b entre lesquelles le corps de la seringues pend.

Comme on peut le voir, les seringues 4 arrivent de manière irrégulière, soit elles sont collées les unes aux autres et orientées de manière différente, ce qui signifie que la collerette ovale 5 peut être perpendiculaire, ou parallèle aux deux parois parallèles 6a et 6b ou encore qu'elles peuvent avoir n'importe quelle orientation intermédiaire. En outre, les seringues 4 peuvent être plus ou moins espacées, en fonction de l'arrivage de ces dernières.

Le dispositif de conditionnement de seringues en nest comprend des moyens de mise au pas 7 des seringues 4 agencés pour séparer et écarter ou resserrer audit pas les seringues amenées, de façon à ce que leur écartement soit régulier. On appelle cette zone la zone de mise au pas.

Dans le cas illustré ici aux figures 2, les moyens de mise au pas comprennent une vis à pas 7 homogène. Il va de soi, que dans certaines formes de réalisations, une vis à pas convergente ou divergente pourra être plus appropriée.

Le dispositif de conditionnement de seringues 4 en nests 1 comprend en outre, une chaîne de transport 8 à maillons 9 présentant ledit pas. La chaîne est en mouvement au moins autour de deux axes 10 et 11 d'entrainement. Lorsque les seringues 4 arrivent sur la bande transporteuse 6, elles sont mises au pas par la vis de mise au pas 7 et ensuite transférées par l'intermédiaire de la roue de transfert 12 et ensuite les seringues sont logées au moins partiellement dans un maillon 8 de la chaîne de transport 9. Les seringues 4 sont donc transportées et mises en mouvement après la zone de mise au pas.

Le dispositif de conditionnement de seringues en nests 1 selon l'invention comporte de plus des premiers moyens de préhension 13 comportant une extrémité liée 14 à un bras d'actionnement (par exemple robotisé) et une extrémité libre 15, par exemple munie de doigts ou de ventouses, permettant la saisie des seringues 4 dans la chaîne de transport 8. Les premiers moyens de préhension 13 prélèvent les seringues 4 de ladite chaîne de transport 8 à une zone dite de prélèvement, qui ne sera pas forcément toujours la même, en fonction de la façon dont les moyens de préhension 13 sont programmés. Les seringues sont donc transportée entre ladite zone de mise au pas et la zone de prélèvement.

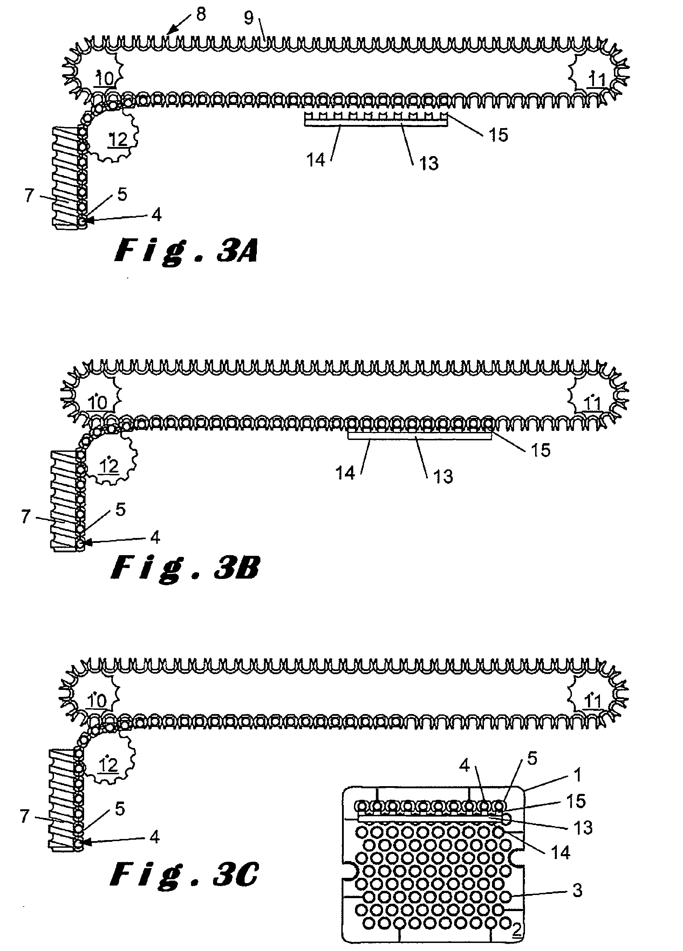

Comme on peut le voir aux figures 3A à 3C, les moyens de préhension 13 prélèvent les seringues 4 en mouvement et le prélèvement comprend, de manière cyclique, une phase d'accostage (figure 3A) des moyens de préhension 13 sur ladite chaîne de transport 8, une phase de prise des seringues 4 (figure 3B) et une phase de déplacement des seringues 4 prélevées vers lesdits nests 1 avec libération des seringues 4 dans le nest 1 susdit (figure 3C).

Les moyens de préhension présentent donc une position de prise (figure 3B) dans laquelle les moyens de préhension saisissent une quantité prédéterminée de seringues. Dans ce cas particulier où la capacité du nest est de 100 seringues (10 lignes de 10 seringues), les moyens de préhension saisissent 10 seringues à la fois. Les moyens de préhension 13 présentent également une position de libération dans laquelle les moyens de préhension 13 relâchent ladite quantité prédéterminée de seringues 4 pour les disposer dans des nests 1 (figure 3C). En outre, les premiers moyens de préhension 13 sont également dans la position de libération pendant la phase d'accostage (figure 3A) de la chaîne de transport 8 (avant la prise des seringues) et les moyens de préhension passent de la position de libération à la position de prise, en mouvement synchronisé avec le mouvement de la chaîne, pour prélever le lot de seringues en mouvement à partir de ladite chaîne.

Comme on le comprendra aisément, l'avantage de ce système de conditionnement de seringues 4 en nests 1 selon l'invention réside dans le fait que les seringues 4 avancent constamment, il n'y a pas d'arrêt de la chaîne 8 ou du convoyeur 6 ou même de la vis de mise au pas 7 pour réaliser la saisie par les moyens de préhension 13, ce qui permet de supprimer les à coup sur le convoyeur ou la bande transporteuse d'entrée 6 qui cause généralement beaucoup de casse.

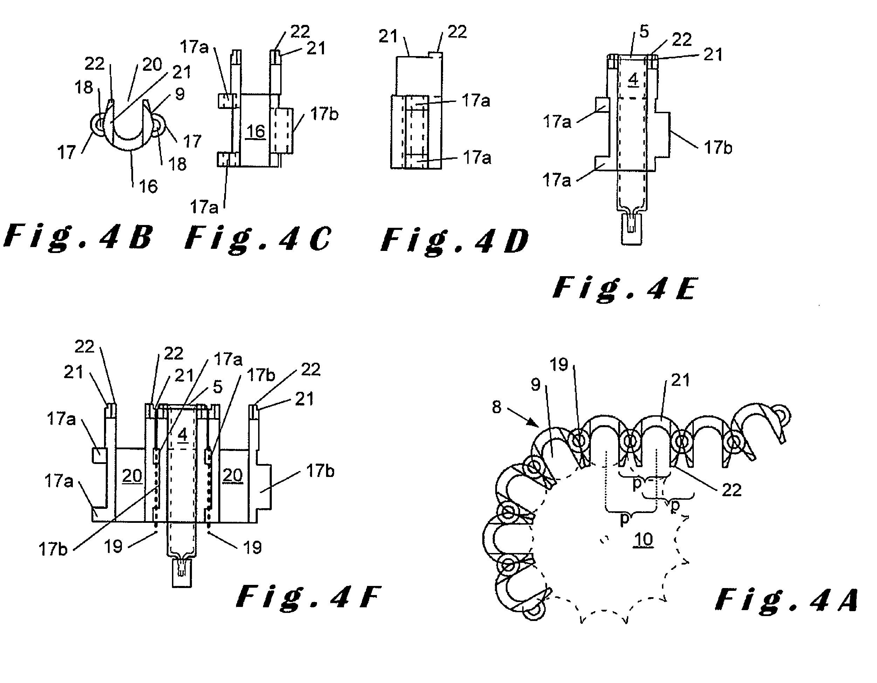

La figure 4A illustre une partie de la chaîne de transport 8 à maillons 9 présentant ledit pas p d'un dispositif de conditionnement, par exemple de seringues 4. La chaîne de transport 8 est entrainée par des axes d'entrainement, qui comprennent par exemple une roue dentée 10 en rotation autour d'un axe, avec lesquelles la chaîne 8 coopère pour entrer en déplacement.

Le maillon 9, comme on peut le voir à la figure 4B comprend une paroi externe 16 d'une section sensiblement identique à la section de la seringue 4 à conditionner ou de tout objet à conditionner. Dans la paroi externe 16, au moins une saillie 17 présentant un orifice 18 central permet de faire passer un axe 19 agissant comme point de pivot entre deux maillons 9 consécutifs. Plus particulièrement, un des deux côtés présente une saillie femelle 17a et l'autre côté présente une saillie mâle 17b (voir figure 4C). Deux maillons 9 consécutifs sont agencés pour que les saillies 17a et 17b présentant un orifice central 18 s'emboitent et pour que l'axe 19 entre les deux maillons 9 serve de point de pivot commun (voir figure 4A).

Comme on le voit clairement à la figure 4B, chaque maillon 8 agencé pour loger au moins une partie d'une seringue 4 comprend une ouverture latérale 20 d'entrée ou de sortie de la seringue 4.

Lorsque le maillon 9 de la chaîne de transport 8 est en mouvement dans le dispositif de conditionnement de seringues selon l'invention, cette ouverture latérale 20 fait face à une seringue 4 mise au pas par lesdits des moyens de mise au pas 7 ou maintenue au pas par ladite roue de transfert 12.

De préférence, ladite ouverture 20 présente une largeur ajustée par rapport à la section de la seringue 4 à loger dans ledit maillon 9. De cette façon, comme on peut le voir à la figure 4E, la seringue est positionnée de manière stable, par un logement de la seringue 4 ajusté dans le maillon 9.

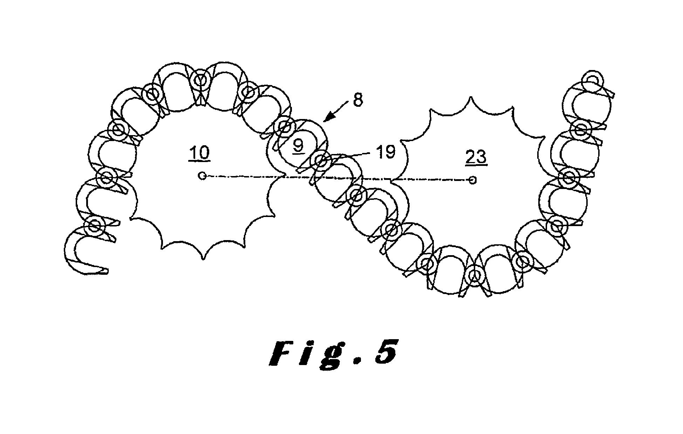

Dans une forme particulière de réalisation, le point de pivot 19 permet aux maillons 9 d'être mobiles l'un par rapport à l'autre de manière unidirectionnelle à partir d'un axe X de chaîne 8 passant par chaque point de pivot 19, c'est-à-dire soit vers l'avant, soit vers l'arrière. A la figure 2B, le point de pivot ne doit permettre aux maillons que d'être mobiles à partir de l'axe X vers l'intérieur du circuit fermé et de pouvoir revenir en position alignée avec l'axe X. Dans une variante, le point de pivot 19 permet aux maillons 9 d'être mobiles l'un par rapport à l'autre de manière bidirectionnelle à partir d'un axe de chaîne 8 passant par chaque point de pivot 19, c'est-à-dire vers l'avant et vers l'arrière, comme on peut le voir à la figure 5. Dans cette figure, le maillon est conçu pour s'enrouler indifféremment sur la face présentant l'ouverture latérale 20 ou sur la face arrière, c'est-à-dire le long de la paroi externe 16 et le dispositif selon l'invention comprend alors au moins un troisième axe d'entrainement 23.

Dans une forme de réalisation avantageuse, chaque maillon 9 présente une surface supérieure 21 munie d'une saillie 22 agencée pour bloquer ladite seringue 4 logée dans ledit maillon 9 et ainsi éviter de manière certaine toute chute et donc toute casse (voir figure 4D). Comme on l'a déjà mentionné, la seringue 4 présentée par les moyens de mise au pas 7 ou par la roue de transfert 12 passe au dessus de ladite saille 22 et redescend au fur et à mesure de son avancement dans ledit maillon 9. Ensuite, la collerette 5 de ladite seringue 4 repose sur la surface supérieure 21 et se trouve alors logée de manière sure.

La figure 4F est une vue de face d'un ensemble de maillon des ladite chaîne de transport.

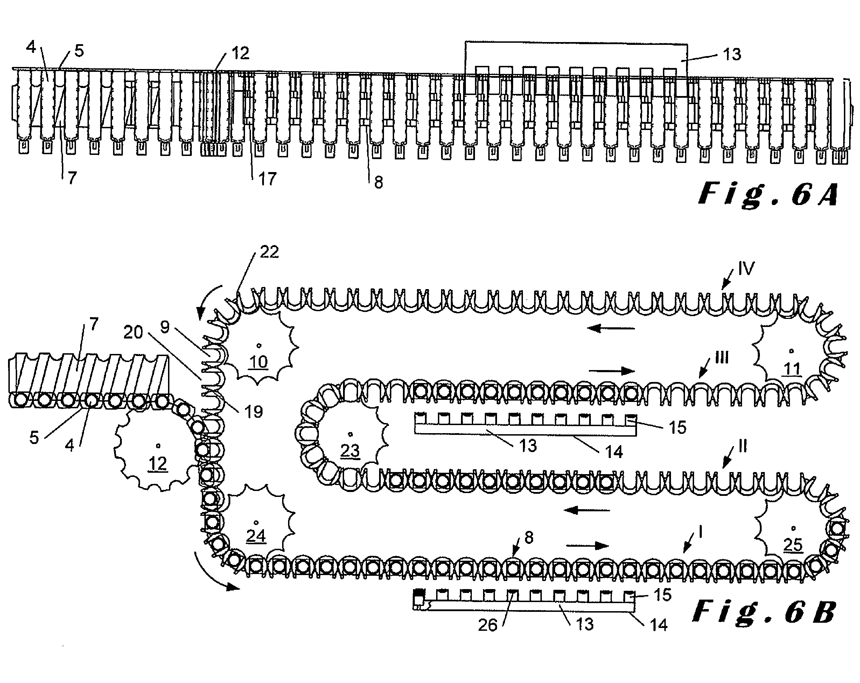

Aux figures 6A et 6B, on peut voir une variante du dispositif de conditionnement de seringues 4 selon l'invention, dans lequel le circuit fermé de la chaîne de transport 8 comprend deux tronçons aller (respectivement I et III ou bien II et IV) et deux tronçons retour (respectivement II et IV ou bien I est III), grâce à la présence d'un quatrième axe d'entrainement 24 et d'un cinquième axe d'entrainement 25. Par les termes tronçons aller (I-III ou II-IV) et tronçons retour (II-IV ou I-III), on entend que la direction d'avancement de la chaîne est inversée. Cette forme de réalisation particulièrement performante comprend des premiers moyens de préhension 13 et des deuxièmes moyens de préhension 13. Les premiers et deuxièmes moyens de préhension 13 sont synchronisés et donc effectuent les étapes de leur cycle à trois phases susdit en même temps. Ici, les moyens de préhension 13 comportent de préférence des ventouses 26 à l'extrémité libre 25 leur permettant à partir d'une réalisation de vide de saisir les seringues 4. Dans cette forme de réalisation, les moyens de préhension 13 saisissent les seringues 4 par l'ouverture 20 dans le maillon 9 qui sert donc également d'orifice de sortie.

Dans une variante, il est également prévu selon l'invention que les moyens de préhension 13 comportent des doigts ou un peigne dont les dents ou les doigts se faufilent entre les seringues 4 et dans lesquels les collerettes 5 reposeront sur deux doigts ou dents adjacentes.

Les moyens de préhension 13 effectuent une légère remontée pour permettre aux seringues 4 de passer au dessus des saillies 22 sur la surface supérieure 21 de chaque maillon 9.

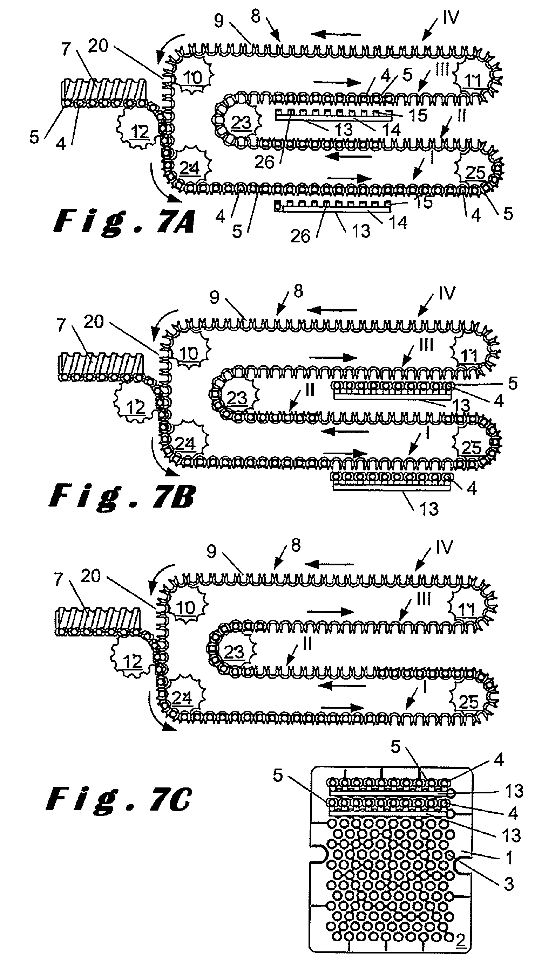

Les figures 7A à 7C sont des vues d'en haut de la forme de réalisation illustrée à la figure 6A ou à la figure 6B, en fonctionnement. La figure 7A représente la première phase du cycle des premiers et des deuxièmes moyens de préhension 13, à savoir la phase d'accostage. La figure 7B illustre la deuxième phase du cycle des premiers de deuxièmes moyens de préhension 13, à savoir la phase de prise. La figure 7C illustre la troisième phase du cycle des premiers et deuxièmes moyens de préhension 13, à savoir la phase de libération des seringues 4 avec conditionnement dans ledit nest 1.

Le dispositif de conditionnement selon l'invention comporte deux tronçons aller et deux tronçons retour sur la chaîne de transport 8 avec la saisie des seringues 4 par les premiers et deuxième moyen de préhension 13 du même coté.

Le dispositif comporte en outre, avantageusement deux convoyeurs parallèles (non illustrés) pour le transport des nests 1.

Dans cette forme avantageuse de réalisation particulièrement rentable et rapide, un même robot commande les premiers et deuxièmes moyens de préhension 13. Les premiers et deuxièmes moyens de préhension 13 accostent respectivement sur un premier I et un troisième tronçon III de la chaîne de transport qui sont synchronisés, c'est-à-dire qu'ils se déplacent à la même vitesse.

Dans cette forme de réalisation, les premiers moyens de préhension de la chaîne de transport prélève un lot de 10 seringues toutes les 20 seringues dans le premier tronçon I et les deuxièmes moyens de préhension prennent en même temps un lot de 10 seringues sur le troisième tronçon III.

Ces deux lots de 10 seringues se déplacent dans le même sens et en phase avec le premier tronçon 1 de la chaîne de transport. De cette manière le robot charge simultanément 20 seringues en deux lots de 10 seringues.

Dans ce cas, les premiers et deuxièmes moyens de préhension 13 chargent du même coté de la chaîne 8. La prise des seringues par la pince est réalisée à titre d'exemple par des ventouses qui tirent les seringues dans une empreinte de guidage de l'extrémité libre 15 desdits moyens de préhension. Ceci est illustré à la figure 7B et représente la phase de prise.

Avant de charger les seringues 4 dans le nest 1, les deux moyens de préhension 13 se rapprochent afin de réaliser l'entre-axe du nest 1.

Ce type de machine est utilisé pour le renestage de nests 1 dont le nombre de rangées est un multiple de 4. En effet les rangées étant trop proches les unes de autres, les moyens de préhension 13 ne peuvent pas charger deux rangées consécutives.

Ceci impose de charger une rangée sur deux. (Les rangées paires ou impaires). Lors du premier chargement, les premiers et deuxièmes moyens de préhension chargent les rangées 1 et 3 et ensuite les rangées 2 et 4. Dans une variante de l'invention, deux robots ayant chacun deux bras auxquels sont respectivement reliés des premiers et deuxièmes moyens de préhension travaillent de manière alternée. Dans ce cas, le fonctionnement global est le même, sauf que, lors du premier chargement, les premiers et deuxièmes moyens de préhension du premier robot chargent les rangées 1 et 3, les premiers et deuxièmes moyens de préhension du deuxième robot charge les rangées 2 et 4, ensuite à nouveau le premier robot charge les rangées 5 et 7 et ainsi de suite.

De cette manière chaque robot dispose d'un temps de cycle correspondant à 40 seringues, soit 4 secondes au total correspondant à 4 lots de 10 seringues par seconde. L'utilisation de ce principe permet de supprimer les arrêts à chaque constitution d'un lot de seringue et permet d'avoir une vitesse linéaire constante à l'entrée des seringues.

L'entraînement de l'ensemble est réalisé en un point, ce qui permet de réaliser une platine par format de seringue. La chaîne de transport est par exemple réalisée au moyen de maillons en plastique injecté. II va de soi que le pas de la chaîne de transport de des moyens de mise au pas ainsi que de l'éventuelle roue de transfert correspondent au pas du nest.

Généralement, le dispositif de conditionnement selon l'invention comporte un dispositif d'éjection (non illustré) de seringues en fin de circuit et de préférence, lesdits moyens de mise au pas 7 des seringues 4, ladite chaîne de transport 8 à maillons 9 présentant ledit pas, ladite roue de transfert 12 et éventuellement ledit dispositif d'éjection sont agencés sur une plate-forme interchangeable afin de pouvoir changer de plate forme rapidement, lorsque les seringues 4 à conditionner ont un diamètre différent.

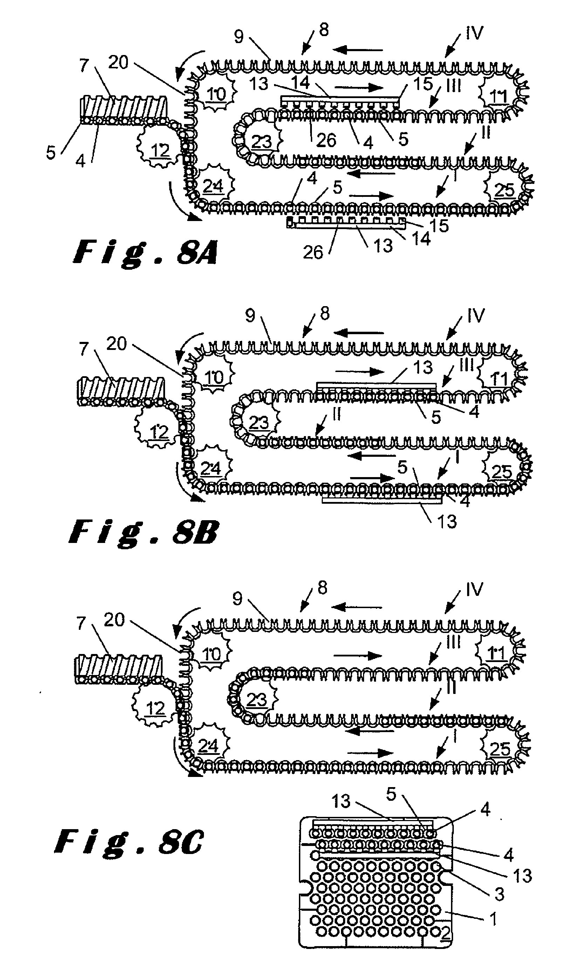

Aux figures 8A à 8C, le dispositif de conditionnement de seringues 4 selon l'invention comporte deux tronçons aller I et III et deux tronçons retour II et IV sur la chaîne de transport 8 avec la saisie des seringues 4 par les premiers et deuxième moyen de préhension 13 du coté opposé.

Le dispositif comporte en outre, avantageusement deux convoyeurs parallèles (non illustrés) pour le transport des nests 1.

Ce dispositif de conditionnement selon l'invention est de préférence conçu pour les nests 1 dont le nombre de rangées est un multiple de deux.

Dans ce cas, les premiers et deuxièmes moyens de préhension (pinces) 13 doivent charger deux lignes contigües. Ceci impose que les moyens de préhension 13 soient à l'extérieur et les seringues 4 à l'intérieur, lorsque les moyens de préhension 14 se rapprochent pour réaliser l'entre-axe du nest 1.

La prise des seringues 4 sur la chaîne de transport sera identique à celle expliquée pour les figures 6 et 7 pour le premier tronçon I par contre la prise des seringues sera sur la face opposée pour le troisième tronçon. Les deuxièmes moyens de préhension du troisième tronçon doivent passer dans la lumière de la partie supérieure du maillon (figure 1).

Pour le reste, l'ensemble du dispositif de conditionnement de seringues selon l'invention est identique à ce qui a été détaillé pour les formes de réalisation précédentes des figures 6 et 7.

Il est bien entendu que la présente invention n'est en aucune façon limitée aux formes de réalisations décrites ci-dessus et que bien des modifications peuvent y être apportées sans sortir du cadre des revendications annexées.

Par exemple, la présente invention se réfère à des seringues, mais il va de soi qu'elle s'applique également à des flacons ou encore à tout type de récipient ou d'objets pour lequel le dispositif de conditionnement peut être utile, par exemple, les flacons stériles d'antibiotiques qui doivent être conditionnées dans des emballages de manière ordonnée et prédisposée ou encore des stylobilles ou des feutres dans des présentoirs.

En outre, on a clairement illustré l'invention en faisant fonctionner deux moyens de préhension de manière synchronisée. Il va de soi que les premiers et deuxièmes moyens de préhension peuvent être décalés exactement, de manière telle que lesdits premiers moyens de préhension passent de la position de libération à la position de prise et saisissent lesdites seringues lorsque lesdits deuxièmes moyens de préhension prélèvent lesdites seringues en mouvement de la chaîne et les libèrent dans le nest.

En outre, on a généralement illustré l'invention avec un accostage sur le premier et le troisième tronçon, il va de soi que ceux-ci peuvent également avoir lieu sur les deuxième et quatrième tronçon ou encore d'autres tronçon lorsqu'il y en a moins ou plus, en fonction de la manière dont le robot qui commande les moyens de préhension est programmé.

## Revendications

1. Dispositif de conditionnement de seringues (4) en nests (1) comprenant :
- des moyens de mise au pas (7) des seringues (4), agencés pour séparer et écarter audit pas les seringues (4) amenées,
- des premiers moyens de préhension (13) desdites seringues (4) mises audit pas, présentant une position de prise dans laquelle les moyens de préhension (13) saisissent une quantité prédéterminée de seringues (4) et une position de libération dans laquelle les moyens de préhension (13) relâchent ladite quantité prédéterminée de seringues (4) pour les disposer dans des nests (1),
**caractérisé en ce qu'**il comprend en outre une chaîne de transport (8) à maillons (9) présentant ledit pas, en circuit fermé et en mouvement au moins autour de deux axes d'entrainement (10,11), agencée pour transporter et mettre en mouvement les seringues (4) provenant des moyens de mise au pas (7), chaque maillon (9) étant agencé pour loger au moins une partie d'une seringue (4), et **en ce que** lesdits premiers moyens de préhension (13) passent de la position de libération à la position de prise, en mouvement synchronisé avec le mouvement de la chaîne (8), pour prélever une quantité prédéterminée de seringues (4) en mouvement à partir de ladite chaîne (8).

2. Dispositif selon la revendication 1, comprenant en outre, une roue de transfert (12) agencée pour charger les seringues (4) mises au pas par lesdits moyens de mise au pas (7) dans ladite chaîne de transport (8).

3. Dispositif selon la revendication 1 ou la revendication 2, dans lequel lesdits maillons (9) de la chaîne (8) sont reliés entre eux par un point de pivot (19) permettant aux maillons (9) d'être mobiles l'un par rapport à l'autre de manière unidirectionnelle à partir d'un axe (X) de chaîne (8) passant par chaque point de pivot (19).

4. Dispositif selon la revendication 1 ou la revendication 2, dans lequel lesdits maillons (9) de la chaîne (8) sont reliés entre eux par un point de pivot (19) permettant aux maillons (9) d'être mobiles l'un par rapport à l'autre de manière bidirectionnelle à partir d'un axe X de chaîne (8) passant par chaque point de pivot (19).

5. Dispositif selon l'une quelconque des revendications 1 à 4, dans lequel chaque maillon (9) présente une section sensiblement identique à la section de la seringue (4) à conditionner.

6. Dispositif selon l'une quelconque des revendications 1 à 5, dans lequel chaque maillon (9) agencé pour loger au moins une partie d'une seringue (4) comprend une ouverture latérale (20) d'entrée ou de sortie de la seringue (4) faisant face à une seringue (4) mise au pas par lesdits des moyens de mise au pas (7).

7. Dispositif selon la revendication 6, dans laquelle ladite ouverture (20') présente une largeur ajustée par rapport à la section de la seringue (4) à loger dans ledit maillon (9).

8. Dispositif selon l'une quelconque des revendications 1 à 7, dans lequel chaque maillon (9) présente une surface supérieure (21) munie d'une saillie (22) agencée pour bloquer ladite seringue (4) logée dans ledit maillon (9).

9. Dispositif selon l'une quelconque des revendications 1 à 8, comprenant en outre, un convoyeur de préférence externe à ladite chaîne de transport (8) en circuit fermé pour le transport des nests (1) à conditionner ou conditionnés.

10. Dispositif selon l'une quelconque des revendications précédentes, dans lequel lesdits moyens de mise au pas (7) sont alimentés par des moyens d'amenée des seringues, en particulier par un convoyeur (6) ou une bande transporteuse.

11. Dispositif selon l'une quelconque des revendications précédentes, comportant des deuxièmes moyens de préhension (13) desdites seringues (4) mises audit pas, présentant une position de prise dans laquelle les deuxièmes moyens de préhension (13) saisissent une quantité prédéterminée de seringues (4) et une position de libération dans laquelle les deuxièmes moyens de préhension (13) relâchent ladite quantité prédéterminée de seringues (4) pour les disposer dans des nests (1), lesdits deuxièmes moyens de préhension (13) passant de la position de libération à la position de prise, en mouvement synchronisé avec le mouvement de la chaîne (8), pour prélever une quantité prédéterminée de seringues (4) en mouvement à partir de ladite chaîne (8).

12. Dispositif selon l'une quelconque des revendications précédentes, dans lequel le nombre d'axes d'entrainement (10, 11, 23, 24, 25) vaut au moins 4 et dans lequel ladite chaîne (8) comprend plus d'un tronçon aller et plus d'un tronçon retour.

13. Dispositif selon la revendication 12, dans lequel lesdits premiers et deuxièmes moyens de préhension (13) sont synchronisés et passent de la position de libération à la position de prise et saisissent lesdites seringues (4) ensemble et les libèrent dans le nest (1) ensemble, lesdits premiers et deuxièmes moyens de préhension (13) étant agencés pour prélever lesdites seringues (4) en mouvement de la chaîne (8), chacun sur un tronçon aller différent ou chacun sur un tronçon retour différent.

14. Dispositif selon l'une des revendications précédentes, comprenant en outre un dispositif d'éjection de seringues (4) en fin de circuit.

15. Dispositif selon l'une quelconque des revendications précédentes, dans lequel lesdits moyens de mise au pas (7) des seringues (4), ladite chaîne de transport (8) à maillons (9) présentant ledit pas, ladite roue de transfert (12) et éventuellement ledit dispositif d'éjection sont agencés sur une plate-forme interchangeable.

16. Procédé de conditionnement de seringues (4) dans des nests comprenant les étapes de:
- amenée et mise au pas des seringues (4) à conditionner dans une zone de mise au pas avec une obtention de seringues (4) mise au pas,
- une préhension des seringues (4) mises au pas à l'aide de moyens de préhension (13),
- un déplacement des seringues (4) prélevées vers lesdits nests (1) dans lesquels lesdites seringues (4) sont à conditionner par les moyens de préhension (13),
**caractérisé en ce qu'**il comprend en outre un transport par une chaîne (8), des seringues (4) entre ladite zone de mise au pas et une zone de prélèvement et **en ce que** le prélèvement par les moyens de préhension (13) est effectué en mouvement et comprend, de manière cyclique, une phase d'accostage des moyens de préhension (13) sur ladite chaîne (8), une phase de prise et une phase de déplacement des seringues (4) prélevées vers lesdits nests (1) avec libération des seringues (4) dans le nest (1) susdit.

## Claims

1. Device for packing syringes (4) in nests (1) comprising:
- means (7) of setting the syringes (4) to the right pitch, arranged to separate and move the brought syringes (4) to the said pitch,
- first means (13) of gripping the said syringes (4) set to the said pitch, having a gripping position in which the gripping means (13) grip a predetermined quantity of syringes (4) and a release position in which the gripping means (13) release the said predetermine quantity of syringes (4) in order to arrange them in nests (1),
**characterised in that** it also comprises a transport chain (8) with links (9) having the said pitch, in closed circuit and moving at least around two drive spindles (10, 11), arranged to transport and move the syringes (4) coming from the pitch-setting means (7), each link (9) being arranged to house at least part of a syringe (4), and **in that** the said first gripping means (13) pass from the release position to the gripping position, in movement synchronised with the movement of the chain (8), in order to take off a predetermined quantity of syringes (4) in movement from the said chain (8).

2. Device according to claim 1, also comprising a transfer wheel (12) arranged to load the syringes (4) set to the right pitch by the said pitch-setting means (7) in the said transport chain (8).

3. Device according to claim 1 or claim 2, in which the said links (9) in the chain (8) are connected together by a pivot point (19) enabling the links (9) to be able to move with respect to one another unidirectionally from an axis (X) of the chain (8) passing through each pivot point (19).

4. Device according to claim 1 or claim 2, in which the said links (9) in the chain (8) are connected together by a pivot point (19) enabling the links (9) to move with respect to one another bidirectionally from an axis X of the chain (8) passing through each pivot point (19).

5. Device according to any one of claims 1 to 4, in which each link (9) has a cross section substantially identical to the cross section of the syringe (4) to be packed.

6. Device according to any one of claims 1 to 5, in which each link (9) arranged to house at least part of a syringe (4) comprises a lateral entry or exit opening (20) for the syringe (4) facing a syringe (4) set to the right pitch by the said pitch-setting means (7).

7. Device according to claim 6, in which the said opening (20') has a width adjusted with respect to the cross section of the syringe (4) to be housed in the said link (9).

8. Device according to any one of claims 1 to 7, in which each link (9) has a top surface (21) provided with a projection (22) arranged to lock the said syringe (4) housed in the said link (9).

9. Device according to any one of claims 1 to 8, also comprising a conveyor preferably external to the said transport chain (8) in closed circuit for transporting nests (1) to be packed or that have been packed.

10. Device according to any one of the preceding claims, in which the said pitch-setting means (7) are supplied by syringe feed means, in particular by a conveyor (6) or a transporter belt.

11. Device according to any one of the preceding claims, comprising second means (13) of gripping the said syringes (4) set to the right pitch, having a gripping position in which the second gripping means (13) grip a predetermined quantity of syringes (4) and a release position in which the second gripping means (13) release the said predetermined quantity of syringes (4) in order to arrange them in nests (1), the said second gripping means (13) passing from the release position to the gripping position, in movement synchronised with the movement of the chain (8), in order to take off a predetermined quantity of syringes (4) in movement from the said chain (8).

12. Device according to any one of the preceding claims, in which the number of driving spindles (10, 11, 23, 24, 25) is at least four and in which the said chain (8) comprises more than one outward section and more than return section.

13. Device according to claim 12, in which the said first and second gripping means (13) are synchronised and pass from the release position to the gripping position and grip the said syringes (4) together and release them in the nest (1) together, the said first and second gripping means (13) being arranged to take off the said moving syringes (4) from the chain (8), each on a different outward section or each on a different return section.

14. Device according to one of the preceding claims, also comprising a device for ejecting syringes (4) at the end of the circuit.

15. Device according to any one of the preceding claims, in which the said means (7) of setting the syringes (4) to the right pitch, the said transport chain (8) with links (9) having the said pitch, the said transfer wheel (12) and possibly the said ejection device are arranged on an exchangeable platform.

16. Method of packing syringes (4) in nests, comprising the steps of:
- bringing and setting to the right pitch syringes (4) to be packed in a pitch-setting zone with obtaining of syringes (4) set to the right pitch,
- gripping of the syringes (4) set to the right pitch by gripping means (13),
- movement of the syringes (4) picked up to the said nests (1) in which
the said syringes (4) are to be packed by the gripping means (13), **characterised in that** it also comprises transportation by a chain (8) of the syringes (4) between the said pitch-setting zone and a picking-up zone and **in that** the picking up by the gripping means (13) is carried out in motion and, in a cyclic manner, comprises a phase of approaching the said chain (8) by the gripping means (13), a gripping phase and a phase of moving the picked-up syringes (4) towards the said nests (1) with the release of the syringes (4) in the aforementioned nest (1).

## Patentansprüche

1. Vorrichtung zum Verpacken von Spritzen (4) in Nestern (1), Folgendes aufweisend:
- Mittel zu Schritteinstellung (7) der Spritzen (4), die eingerichtet sind, um die zugeführten Spritzen (4) zu trennen und zu beabstanden,
- erste Mittel (13) zum Ergreifen der im Schritt eingestellten Spritzen (4), die eine Aufnahmeposition aufweisen, in der die Greifmittel (13) eine vorbestimmte Menge von Spritzen (4) erfassen, und eine Freigabeposition, in der die Greifmittel (13) die vorbestimmte Menge von Spritzen (4) freigeben, um sie in Nestern (1) anzuordnen,
**dadurch gekennzeichnet, dass** sie ferner eine Transportkette (8) mit Gliedern (9) aufweist, die den Schritt aufweisen, im geschlossenen Kreislauf und um mindestens zwei Antriebsachsen (10, 11) in Bewegung, die eingerichtet ist, um die Spritzen (4), die von den Mitteln zur Schritteinstellung (7) kommen, zu transportieren und in Bewegung zu versetzen, wobei jedes Glied (9) eingerichtet ist, um mindestens einen Teil einer Spritze (4) aufzunehmen, und wobei die ersten Greifmittel (13) von der Freigabeposition zu der Aufnahmeposition in einer Bewegung übergehen, die mit der Bewegung der Kette (8) synchronisiert ist, um eine vorbestimmte Menge von Spritzen (4) in Bewegung ausgehend von der Kette (8) zu entnehmen.

2. Vorrichtung nach Anspruch 1, die ferner ein Übertragungsrad (12) aufweist, das eingerichtet ist, um die Spritzen (4), die von den Mitteln zur Schritteinstellung (7) eingestellt wurden, in die Transportkette (8) zu laden.

3. Vorrichtung nach Anspruch 1 oder Anspruch 2, bei der die Glieder (9) der Kette (8) untereinander durch einen Schwenkpunkt (19) verbunden sind, der es den Gliedern (9) erlaubt, zueinander in eine Richtung ausgehend von einer Achse (X) der Kette (8), die durch jeden Schwenkpunkt (19) verläuft, beweglich zu sein.

4. Vorrichtung nach Anspruch 1 oder Anspruch 2, bei der die Glieder (9) der Kette (8) untereinander durch einen Schwenkpunkt (19) verbunden sind, der es den Gliedern (9) erlaubt, zueinander in zwei Richtungen ausgehend von einer Achse (X) der Kette (8), die durch jeden Schwenkpunkt (19) verläuft, beweglich zu sein.

5. Vorrichtung nach einem der Ansprüche 1 bis 4, bei der jedes Glied (9) einen Querschnitt aufweist, der im Wesentlichen mit dem Querschnitt der zu verpackenden Spritze (4) identisch ist.

6. Vorrichtung nach einem der Ansprüche 1 bis 5, bei der jedes Glied (9), das eingerichtet ist, um mindestens einen Teil einer Spritze (4) aufzunehmen, eine seitliche Eingangs- oder Ausgangsöffnung (20) der Spritze (4) aufweist, die einer Spritze (4), die von den Mitteln zur Schritteinrichtung (7) eingerichtet wurde, gegenüberliegt.

7. Vorrichtung nach Anspruch 6, bei der die Öffnung (20') eine Breite aufweist, die in Bezug zu dem Querschnitt der Spritze (4), die in dem Glied (9) aufzunehmen ist, eingestellt ist.

8. Vorrichtung nach einem der Ansprüche 1 bis 7, bei der jedes Glied (9) eine obere Fläche (21) aufweist, die mit einem Vorsprung (22) versehen ist, der eingerichtet ist, um die Spritze (4), die in dem Glied (9) aufgenommen ist, zu blockieren.

9. Vorrichtung nach einem der Ansprüche 1 bis 8, die ferner einen Förderer aufweist, der vorzugsweise außerhalb der Transportkette (8) in geschlossenem Kreislauf für den Transport der Nester (1) die zu verpacken sind oder verpackt wurden, liegt.

10. Vorrichtung nach einem der vorhergehenden Ansprüche, bei der die Mittel zum Einstellen im Schritt (7) von Zuführmitteln der Spritzen versorgt werden, insbesondere von einem Förderer (6) oder von einem Förderband.

11. Vorrichtung nach einem der vorhergehenden Ansprüche, die zweite Greifmittel (13) der im Schritt eingestellten Spritzen (4) aufweist, die eine Greifposition aufweisen, in der die zweiten Greifmittel (13) eine vorbestimmte Menge von Spritzen (4) erfassen, und eine Freigabeposition, in der die zweiten Greifmittel (13) die vorbestimmte Menge von Spritzen (4) freigeben, um sie in Nestern (1) anzuordnen, wobei die zweiten Greifmittel (13) von der Freigabeposition zu der Aufnahmeposition in einer Bewegung übergehen, die mit der Bewegung der Kette (8) synchronisiert ist, um eine vorbestimmte Menge von Spritzen (4) in Bewegung ausgehend von der Kette (8) zu entnehmen.

12. Vorrichtung nach einem der vorhergehenden Ansprüche, bei der die Anzahl von Antriebsachsen (10, 11, 23, 24, 25) mindestens gleich 4 ist, und bei der die Kette (8) mehr als einen Hinlauf- und einen Rücklaufabschnitt aufweist.

13. Vorrichtung nach Anspruch 12, bei der die ersten und zweiten Greifmittel (13) synchronisiert sind und von der Freigabeposition auf die Aufnahmeposition übergehen und die Spritzen (4) gemeinsam erfassen und sie gemeinsam in dem Nest (1) freigeben, wobei die ersten und zweiten Greifmittel (13) eingerichtet sind, um die Spritzen (4) in Bewegung von der Kette (8) zu entnehmen, jeweils auf einem unterschiedlichen Hinlaufabschnitt oder jeweils auf einem unterschiedlichen Rücklaufabschnitt.

14. Vorrichtung nach einem der vorhergehenden Ansprüche, die ferner eine Auswurfvorrichtung von Spritzen (4) am Kreislaufende aufweist.

15. Vorrichtung nach einem der vorhergehenden Ansprüche, bei der die Mittel zum Schritteinstellen (7) der Spritzen (4), die Transportkette (8) mit Gliedern (9), die den Schritt aufweisen, das Übertragungsrad (12) und eventuell die Auswurfvorrichtung auf einer austauschbaren Plattform eingerichtet sind.

16. Verpackungsverfahren von Spritzen (4) in Nestern, das die folgenden Schritte aufweist:
Zuführen und Schritteinstellen der zu verpackenden Spritzen (4) in einer Zone zum Schritteinstellen mit Erzielen von Spritzen (4), die im Schritt eingestellt sind,
- Ergreifen der Spritzen (4), die im Schritt eingestellt sind, mit Hilfe von Greifmittel (13),
- eine Verlagerung der entnommenen Spritzen (4) zu den Nestern (1) in welchen die Spritzen (4) von den Greifmittel (13) zu verpacken sind,
**dadurch gekennzeichnet, dass** es ferner einen Transport der Spritzen (4) durch eine Kette (8) zwischen der Zone zum Schritteinstellen und einer Entnahmezone aufweist, und dass das Entnehmen durch die Greifmittel (13) in Bewegung erfolgt und zyklisch eine Andockphase der Greifmittel (13) auf der Kette (8), eine Aufnahmephase und eine Verlagerungsphase der entnommenen Spritzen (4) zu den Nestern (1) mit Freigabe der Spritzen (4) in dem Nest (1) ausgeführt wird.
